(19) Europäisches Patentamt
European Patent Office
Office européen des brevets

(11) Veröffentlichungsnummer: **0 035 733**
**B1**

(12) # EUROPÄISCHE PATENTSCHRIFT

(45) Veröffentlichungstag der Patentschrift: **14.09.83**

(51) Int. Cl.³: **C 07 C121/80**, C 07 C120/00, A 61 K 31/275

(21) Anmeldenummer: **81101498.4**

(22) Anmeldetag: **03.03.81**

(54) **Neue 1-(Acylamino-aryloxy-)2-hydroxy-3-alkinyl-aminopropane und Verfahren zu ihrer Herstellung.**

(30) Priorität: **08.03.80 DE 3009047**

(43) Veröffentlichungstag der Anmeldung: **16.09.81 Patentblatt 81/37**

(45) Bekanntmachung des Hinweises auf die Patenterteilung: **14.09.83 Patentblatt 83/37**

(84) Benannte Vertragsstaaten: **AT BE CH DE FR IT LI LU NL SE**

(56) Entgegenhaltungen:
**CH A 546 224**
**CH A 546 227**
**DE A 2 309 887**
**DE A 2 403 809**

**Die Akte enthält technische Angaben, die nach dem Eingang der Anmeldung eingereicht wurden und die nicht in dieser Patentschrift enthalten sind.**

(73) Patentinhaber: **C.H. BOEHRINGER SOHN**
**Postfach 200**
**D-6507 Ingelheim am Rhein (DE)**

(72) Erfinder: **Köppe, Herbert, Dr.**
**Neuweg 72**
**D-6507 Ingelheim/Rhein (DE)**
Erfinder: **Kummer, Werner, Dr.**
**Georg-Scheuing-Strasse 15**
**D-6507 Ingelheim/Rhein (DE)**
Erfinder: **Stähle, Helmut, Dr.**
**Rotweinstrasse 23**
**D-6507 Ingelheim/Rhein (DE)**
Erfinder: **Muacevic, Gojko, Dr.**
**In der Dörrwiese 13**
**D-6507 Ingelheim/Rhein (DE)**
Erfinder: **Traunecker, Werner, Dr.**
**Birkenweg 1**
**D-6531 Münster-Sarmsheim (DE)**

**EP 0 035 733 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Neue 1-(Acylamino-aryloxy-) 2-hydroxy-3-alkinyl-aminopropane und Verfahren zu ihrer Herstellung

Die Erfindung betrifft neue Verbindungen der Formel

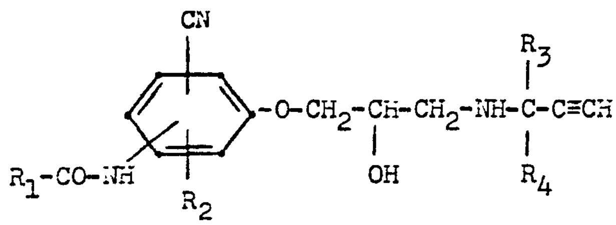

(I)

in der

$R_1$ einen geraden oder verzweigten Alkylrest mit 4 bis 20 C-Atomen bedeutet

$R_2$ ein Wasserstoff- oder Halogenatom, eine Alkyl- oder Alkoxygruppe mit 1 bis 4 C-Atomen oder die zweiwertigen Gruppen $—CH = CH—CH = CH—$ oder $(—CH_2—)_n$ (n = ganze Zahl von 3 bis 5) mit Bindung der freien Valenzen in o-Stellung zueinander

$R_3$ Wasserstoff oder einen Alkylrest mit 1 bis 3 C-Atomen

$R_4$ einen Alkylrest mit 1 bis 3 C-Atomen oder zusammen mit $R_3$ die Gruppe $(—CH_2—)_p$ (p = ganze Zahl von 4 bis 6),

ihre Säureadditionssalze, die Verwendung dieser Verbindungen in Arzneimitteln sowie ihre Herstellung.

Die neuen Verbindungen können auf folgende Weise hergestellt werden :

a) Umsetzung einer Verbindung der allgemeinen Formel

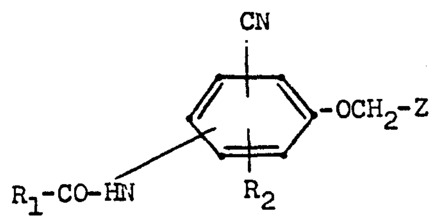

(II)

in der $R_1$ und $R_2$ wie in Formel I definiert sind und Z die Gruppe

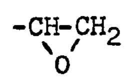

oder $—CHOH—CH_2—Hal$ (Hal = Halogen) bedeutet, mit einem Amin der allgemeinen Formel

$$NH_2—CR_3R_4—C \equiv CH \qquad (III)$$

in der $R_3$ und $R_4$ die in Formel I angegebene Bedeutung haben.

b) Hydrolyse eines Oxazolidin-Derivats der allgemeinen Formel

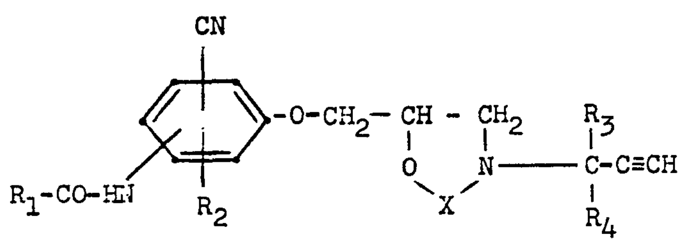

(IV)

in der $R_1$ bis $R_4$ wie in Formel I definiert sind und X eine $—CO—$, $—CH_2—$ oder $—CH—$ niederalkyl-Gruppe bedeutet, beispielsweise mit Natron- oder Kalilauge in Wasser oder in einer Alkohol-Wasser-Mischung.

c) Umsetzung einer Verbindung der allgemeinen Formel V

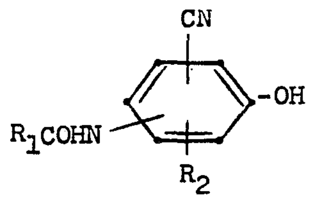

(V)

in welcher $R_1$ und $R_2$ die oben genannte Bedeutung haben, bzw. eines Salzes dieses Phenols, mit einem Azetidinolderivat der allgemeinen Formel IV

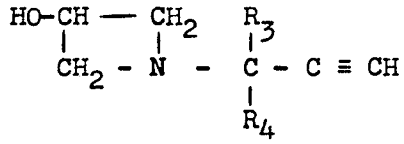

(VI)

in welcher $R_3$ und $R_4$ die oben genannte Bedeutung haben, in wasserfreiem Medium.

Die Oxazolidinone der Formel IV (d. h. Verbindungen, bei denen X = CO darstellt) sind beispielsweise ausgehend von den Epoxiden der Formel II herstellbar, indem man letztere mit einem (aus Chlorameisensäureäthylester und einem Amin der Formel III darstellbaren) Urethan der Formel VII

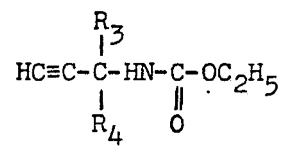

(VII)

in der $R_3$ und $R_4$ die oben bezeichneten Bedeutungen haben umsetzt.

Die Ausgangsphenole der allgemeinen Formel V und die Azetidinole der allgemeinen Formel VI können nach literaturbekannten Methoden (letztere s. beispielsweise Chem. pharm. Bull. (Japan), Voll. 22 (7), 1974, Seite 1490) hergestellt werden.

Die erfindungsgemäßen Verbindungen besitzen ein asymmetrisches C-Atom an der CHOH-Gruppe und kommen daher als Racemat wie auch in Form der optischen Antipoden vor. Letztere können außer durch Racematentrennung mit üblichen Hilfssäuren wie Dibenzoyl- (bzw. Di-p-Toluyl-) D-Weinsäure oder D-3-Bromcampher-8-sulfonsäure auch durch Einsetzen von optisch aktiven Ausgangsmaterial erhalten werden.

Die erfindungsgemäßen 1-Phenoxy-2-hydroxy-3-alkinyl-aminopropane der allgemeinen Formel I können in üblicher Weise in ihre physiologisch verträglichen Säureadditionssalze überführt werden. Geeignete Säuren sind beispielsweise Salzsäure, Bromwasserstoffsäure, Schwefelsäure, Methansulfonsäure, Maleinsäure, Essigsäure, Oxalsäure, Milchsäure, Weinsäure oder 8-Chlortheophyllin.

Die Verbindungen der allgemeinen Formel I bzw. deren physiologisch verträglichen Säureadditionssalze haben im Tierversuch an Meerschweinchen wertvolle therapeutische, insbesondere β-adrenolytische Eigenschaften gezeigt und können daher beispielsweise zur Behandlung oder Prophylaxe von Erkrankungen der Herzkranzgefäße und zur Behandlung von Herzarrhythmien, insbesondere von Tachycardien, in der Humanmedizin eingesetzt werden. Auch die blutdrucksenkenden Eigenschaften der Verbindungen sind therapeutisch interessant. Die Verbindungen sind therapeutisch interessant. Die Verbindungen haben gegenüber bekannten β-Rezeptorenblockern, z. B. dem strukturverwandten Handelsprodukt 1-(2-Acetyl-4-butyroylaminophenoxy-2-hydroxy-3-isopropyl-aminopropan (Acebutolol) den Vorteil beträchtlich verminderter Toxizität, besserer aludrinantagonistischer Wirkung und hervorragender Organselektivität. Die Messung dieser Parameter erfolgte dabei nach den folgenden Modellvorschriften :

1. Hemmung der Isoprenalintachycardie
(aludrinantagonistische Wirkung)

Methode : Hemmung der tachycarden Reaktion auf eine Standard-Dosis Isoprenalin und Einfluß auf die basale Herzfrequenz durch steigende i. v. Dosen eines β-Adrenolytikums.

Tiermaterial : Meerschweinchen beiderlei Geschlechts mit Körpergewichten von 270 bis 350 g, Gruppenhaltung, Standard-Kost und Wasser bis zum Versuchsbeginn ad libitum. 16 Stunden vor dem Versuchsanfang Futterentzug.

Narkose : Äthylurethan 1,75 g/kg als 20 %ige Lösung intrapertoneal, gegebenenfalls wurde nachinjiziert.

Präparation : Kanülierung einer Vena jugularis exerior für intravenöse Injektionen : Einbinden einer

Trachealkanüle und künstliche Beatmung ; subcutane Nadelektroden zur Aufnahme des EKG, in der Regel Extremitätenableitung II, Registriergeschwindigkeit 25 mm/sec ; Rektalthermometer zur Kontrolle der Körpertemperatur, die mit einer Wärmelampe (Infrarotstrahler) auf 34 bis 35 °C mit Hilfe einer elektronischen Automatikeinrichtung konstant gehalten wird.

Versuchsablauf : Die Herzfrequenz wird durch Auszählen der R-Zacken im EKG bestimmt, jeweils aus einer 3 bis 4 sec. langen Registrierzeit. Etwa 30 min nach der Präparation wird im Abstand von 2 min 5 mal die normale Herzfrequenz gemessen 1 µg/kg Isoprenalin i. v. injiziert und danach 3 min lang alle 30 sec die Herzfrequenz erneut registriert. Die Isoprenalininjektionen werden während des ganzen Versuchs im Abstand von 30 min wiederholt. Bleibt die Spontanfrequenz etwa konstant und ist die tachycarde Reaktion auf die ersten 2 bis 3 Isoprenalin-Gaben gleichmäßig, dann wird 15 min nach der letzten und 15 min vor der nächsten Isoprenalin-Reaktion die erste Dosis der Prüfsubstanz i. v. injiziert. Weitere in geometrischer Reihe aufsteigende Dosen der Prüfsubstanz folgen in Abständen von 60 min. bis eine markante Hemmung der Isoprenalin-Tachycardie erreicht ist.

2. Prüfung auf Cardioselektivität am wachen Meerschweinchen

Prinzip : Nach der Methode von D. Dunlop und R. G. Shanks (Brit. J. Pharmacol. *32,* 201 1968) werden wache Meerschweinchen einer tödlichen Dosis eines Histaminaerosols ausgesetzt. Durch Vorbehandlung mit Isoprenalin werden die Tiere vor der letalen Wirkung des Histamins geschützt. Ein β-Adrenolytikum hebt die Isoprenalinwirkung auf, so daß der Schutz vor dem Histaminbronchospasmus verloren geht, falls es sich um eine nicht cardioselektive Substanz handelt. Zeigt eine am Herzen wirksame β-adrenolytische Substanz in diesem Test keinen Antagonismus gegen Isoprenalin, dann kann Cardioselektivität für sog. $\beta_1$-Rezeptoren) angenommen werden.

Tiermaterial : Meerschweinchen beiderlei Geschlechts (6 Tiere pro Dosis), mit 350 bis 400 g Körpergewicht, Gruppenhaltung. Standard Futter und Wasser bis zum Versuchsbeginn ad libitum. 16 Stunden vor dem Versuchsanfang Futterentzug.

Versuchsverlauf : Gruppen von je 6 Tieren (3 männliche + 3 weibliche) werden s.c. mit 5 oder mehr verschiedenen Dosen des β-adrenolytikums behandelt. Fünfzehn Minuten später erhalten sie 0,1 mg/kg Isoprenalin contralateral s.c. injiziert. Nach weiteren 15 Minuten werden die Tiere in die zylindrische Kammer von 2 Liter Inhalt gesetzt, 45 Sekunden lang einem wäßrigen Histaminaerosol (1,25 %ig) ausgesetzt und anschließend wird die Mortalität ausgewertet.

Auswertung : Die Letalität wird gegen den Logarithmus der Dosis aufgetragen und die LD 50 nach J. Litchfield und F. Wilcoxon (J. Pharmacol. exp. Theram. *96,* 99-113, 1949) ermittelt. Mit der LD 50 aus diesem Versuch und der cardialen ED 50 aus dem Versuch der Hemmung der Isoprenalintachycardie (narkot. Meerschweinchen) wird ein Selektivitätsquotient (LD 50/ED 50) gebildet. Eine Substanz wird als cardioselektiv angesehen, wenn der Quotient größer als 1 ist.

Als wertvoll haben sich dabei insbesondere solche Verbindungen der allgemeinen Formel I herausgestellt, bei denen $R_3$ und $R_4$ jeweils eine Methylgruppe darstellen (substituierte p-Acylamino-1-phenoxy-3-(2-methylbutinyl)-3-amino-2-2-propanole). Besonders wertvoll ist insbesondere das 1-(2-Cyano-4-n-hexano-ylamino) phenoxy-3-(2-methylbutinyl-3-amino-2)-2-propanol bzw. seine Salze. Diese Verbindung hat z. B. eine gegenüber dem Acebutolol etwa vierzigfach stärkere aludrinantagonistische Wirkung.

Die Einzeldosis der erfindungsgemäßen Substanzen liegt bei 1 bis 300 mg, vorzugsweise 5 bis 100 mg (oral) bzw. 1 bis 20 mg (parenteral).

Die erfindungsgemäßen Wirkstoffe können in die üblichen galenischen Anwendungsformen, wie Tabletten, Dragées, Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung eines Depoteffekts wie Carboxypolymethylen, Carboxymethylcellulose, Celluloseacetatphthalat, oder Polyvinylacetat erhalten werden.

Die Tabletten können auch aus mehreren Schichten bestehen. Entsprechend können Dragées durch Überziehen von analog den Tabletten hergestellten Kernen mit üblicherweise in Dragéeüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Dragéehülle zur Erzielung eines Depoteffekts aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker, sowie ein geschmackverbesserndes Mittel, z. B. Aromastoffe, wie Vanillin oder Orangenextrakt, enthalten.

Sie können außerdem Suspendierhilfsstoffe oder Dickungsmittel, wie Natriumcarboxymethyl-cellulose, Netzmittel, beispielsweise Kondensationsprodukte von Fettalkoholen mit Äthylenoxid, oder Schutzstoffe wie p-Hydroxybenzoate, enthalten.

Injektionslösungen werden in üblicher Weise, z. B. unter Zusatz von Konservierungsmitteln, wie p-Hydroxybenzoaten, oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen der dafür vorgesehenen Wirkstoffe bzw. Wirkstoffkombinationen mit üblichen Trägermitteln, wie Neutralfetten oder Polyäthylenglykol bzw. dessen Derivaten, herstellen.

Die erfindungsgemäßen Verbindungen sind auch für die Kombination mit anderen pharmakodynamisch wirksamen Stoffen wie z. B. Coronardilatatoren, Symphathicomimetica, Herzglykosiden oder Tranquilizer geeignet.

Die folgenden Beispiele erläutern die Erfindung, ohne sie zu beschränken :

## A. Herstellungsbeispiele

### Beispiel 1

1-(2-Cyano-4-α-methylpentanoylaminophenoxy)-3-(2-methylbutinyl-3-amino-2)-2-propanol

14 g (0,048 Mol) 1-(2-Cyano-4-α-methylpentanoylaminophenoxy)-2,3-epoxypropan werden in 100 ml Äthanol gelöst und 12,5 g (0,15 Mol) 2-Methylbutin-3-amin-2 zugegeben. Nach einstündigem Sieden unter Rückfluß wird das Lösungsmittel i. V. abdestilliert.

Der Rückstand wird mit verdünnter HCl angesäuert und zweimal mit Äther ausgeschüttelt. Die wäßrige Phase wird mit $NH_4OH$ alkalisch gestellt und die ausfallende ölige Base in Essigester aufgenommen. Die organische Phase wird mit Wasser gewaschen, über $MgSO_4$ getrocknet und filtriert. Nach Abdestillieren des Lösungsmittels wird der Rückstand über eine Kieselgelsäule gereinigt (Essigester/Isopropanol/$NH_4OH$ 7 : 3 : 1). Die Fraktionen der Reinsubstanz ergeben nach Abdestillieren 7,6 g Base, die in Äther aufgenommen und mit Wasser gewaschen wird. Nach Trocknung wüber $MgSO_4$ und Aktivkohle wird abgesaugt und der Äther abdestilliert. Es verbleibt eine nicht kristallisierende, viskose Base. DC einheitlich. Ausbeute : 7,4 g.

### Beispiel 2

1-(2-Cyano-4-n-hexanoylaminophenoxy)-3-(2-methyl-butinyl-3-amino-2)-2-propanol

41,3 g (0,18 Mol) 1-(2-Cyano-4-n-hexanoylaminophenoxy)-2,3-epoxypropan werden in 400 ml Äthanol gelöst und nach Zugabe von 33,2 g (0,4 Mol) 2-Methylbutin-3-amin-2 1,5 Stunden zum Sieden unter Rückfluß erhitzt. Nach Abdestillieren des Lösungsmittels wird der dunkle Rückstand aus 250 ml Acetonitril umkristallisiert. Nach Absaugen des rohen Kristallisats wird nochmals aus 250 ml Acetonitril unter Zugabe von Aktivkohe umkristallisiert. Es wurden 16,9 g reinweiße Kristalle gewonnen. Fp. : 119 bis 122 °C.

### Beispiel 3

1-(2-Cyano-4-α-äthylbutyroylaminophenoxy)-3-(2-methylbutinyl-3-amino-2)-2-propanol-hydrochlorid

14 g (0,049 Mol) 1-(2-Cyano-4-α-äthyl-butyroylaminophenoxy)-2,3-eposypropan werden in 100 ml Äthanol gelöst und nach Zugabe von 12,5 g (0,15 Mol) 2-Methyl-butin-3-amin-2 eine Stunde unter Rückfluß zum Sieden erhitzt. Das Lösungsmittel wird i. V. eine Stunde unter Rückfluß zum Sieden erhitzt. Das Lösungsmittel wird i. V. abdestilliert, der verbleibende Rückstand wie in Beispiel 1 über eine Kieselgelsäule gereinigt. Das so gewonnene Reinprodukt wird in 50 ml Acetonitril gelöst, äthanolische

HCl zugegeben und durch Zugabe von Äther das Hydrochlorid zur Kristallisation gebracht. Nach Isolierung und Trocknung werden 4,5 g reinweiße Kristalle gewonnen. Fp. : 202 bis 205 °C.

Beispiel 4

1-[2-Cyano-4-(3,3-dimethylbutyroylamino)-phenoxy]-3-(2-methylbutinyl-3-amino-2)-2-propanol-hydrochlorid

14 g (0,049 Mol) 1-[2-Cyano-4-(3,3-dimethylbutyroyl-amino)-phenoxy]-2,3 epoxypropan werden wie in Beispiel 3 nach Lösen in 100 ml Äthanol mit 12,5 g (0,15 Mol) 2-Methylbutin-3-amin-2 der Aminolyse in der Siedehitze unterworfen. Nach Abdestillieren des Lösungsmittels wird der Rückstand mit verdünnter HCl digeriert, wobei sich der größte Teil löst. Zur Abtrennung nichtbasischer Anteile wird mit Äther extrahiert und die wäßrige Phase mit $NH_4OH$ alkalisch gestellt. Die sich abscheidende Base wird in Essigester aufgenommen. Dieser wird nach Waschen mit $H_2O$ über $MgSO_4$ getrocknet und abdestilliert. Der verbleibende Rückstand wird wie in Beispiel 1 über eine Kieselgelsäule gereinigt. Nach Aufarbeitung der Fraktionen verbleibt ein viskoser Rückstand, der in Äthanol gelöst und mit äthanolischer HCl angesäuert wird. Das Lösungsmittel wird i. V. abdestilliert, der Rückstand in wenig Acetonitril gelöst und alkoholische HCl zugegeben. Nach Zugabe von Äther fällt das Hydrochlorid kristallin aus. Es werden 5,8 g reinweißes Kristallisat isoliert. Fp. : 186 bis 188 °C.

Beispiel 5

1-(2-Cyano-4-n-octanoylaminophenoxy)-3-(2-methyl-butinyl-3-amino-2)-propanol

10 g (0,03 Mol) 1-(2-Cyano-4-n-octanoylaminophonoxy)-2,3-epoxypropan wurden in 80 ml Äthanol gelöst und nach Zugabe von 10 ml 2-Methylbutin-3-amin-2 80 Minuten unter Rückfluß zum Sieden erhitzt. Nach Erkalten wird das Lösungsmittel abdestilliert und der kristalline Rückstand aus Acetonitril umkristallisiert. Das reinweiße Kristallisat wird nochmals aus Acetonitril umkristallisiert, wobei 3,7 g reine Base gewonnen werden. Fp. : 110 bis 112 °C.

Beispiel 6

1-(2-Cyano-4-n-hexanoylaminophenoxy)-3-(1-äthinyl-cyclohexylamino)-propanol-2

10,4 g (0,033 Mol) 1-(2-Cyano-4-n-hexanoylaminophenoxy)-3-chlorpropanol-2 werden in 80 ml Äthanol mit 12,3 g (0,1 Mol) 1-Äthinylcyclohexylamin 3,5 Stunden unter Rückfluß zum Sieden erhitzt. Nach Verdampfen des Lösungsmittels i. V. wird der verbleibende Rückstand mit verdünnter HCl digeriert, zweimal mit Äther ausgeschüttelt, danach abgeschiedenes öliges Produkt und wäßrige Phase mit $NH_4OH$ versetzt und mit Essigester extrahiert. Die organische Phase wird mit $H_2O$ gewaschen, über $MgSO_4$ getrocknet und i. V. zur Trockene eingeengt.

Der feste Rückstand wird zweimal aus Acetonitril umkristallisiert. Ausbeute : 4,2 g farblose Kristalle. Fp. : 125 bis 127 °C.

Beispiel 7

1-(2-Cyano-6-n-hexanoylaminophenoxy)-3-(2-methyl-butinyl-3-amino-2)-2-propanol-hydrochlorid

10 g (0,031 Mol) 1-(2-Cyano-6-n-hexanoylaminophenoxy)-3-chlor-2-propanol werden in 80 ml Äthanol gelöst und nach Zugabe von 12,6 ml (0,12 Mol) 2-Methyl-butin-3-amin-2 zwei Stunden unter Rückfluß gekocht. Nach Aufarbeitung wie in Beispiel 6 wird die wäßrige saure Phase mit NaOH alkalisch gestellt, die ausfallende ölige Base in Essigester aufgenommen und wie in oben beschriebener Weise aufgearbeitet. Die rohe Base wird über eine Kieselgelsäule gereinigt. Das gereinigte Präparat wird in Acetonitril gelöst, mit alkoholischer HCl angesäuert und durch Zugabe von Äther zur Kristallisation gebracht. Das Hydrochlorid wird aus Acetonitril unter Zugabe von Äther nochmals umkristallisiert. Ausbeute : 2,1 g Fp. : 113 bis 134 °C.

Beispiel 8

1-(2-Cyano-6-n-heptanoylaminophenoxy)-3-(3-äthylpentinyl-4-amino-3)-2-propanol-hydrochlorid

16 g (0,047 Mol) 1-(2-Cyano-6-n-heptanoylaminophenoxy)-3-chlor-2-propanol werden nach Lösen in 80 ml Äthanol in Gegenwart von 26 ml (0,2 Mol) 3-Äthylpentin-4-amin-3 zwei Stunden unter Rückfluß zum Sieden erhitzt. Nach Aufarbeitung wie in Beispiel 6 wird die rohe Base über eine Kieselgelsäule gereinigt. Nach Vereinigung der einheitlichen Fraktionen wird die Base durch Abdestillieren des Lösungsmittelgemisches gewonnen. Sie wird in verdünnter HCl aufgenommen und mit Äther geschüttelt. Der dabei ausfallende kristalline Körper wird isoliert und das Acetonitril umkristallisiert. Ausbeute : 2,7 g. Fp. : 188 bis 189 °C.

Beispiel 9

1-(2-Cyano-6-n-pentanoylaminophenoxy)-3-(2-methyl-butin-3-amino-2)-2-propanol-hydrochlorid

11 g (0,036 Mol) 1-(2-Cyano-6-n-pentanoyl-aminophenoxy)-3-chlor-2-propanol werden nach Lösen in 80 ml Äthanol und Zugabe von 11,6 g (0,11 Mol) 2-Methyl-butin-3-amin-2 drei Stunden zum Sieden unter Rückfluß erhitzt. Die Aufarbeitung und Säulentrennung erfolgt wie in Beispiel 8. Der einheitliche basische Rückstand der Säulenfraktionen wird in wenig Acetonitril gelöst, mit ätherischer HCl angesäuert und durch Zugabe von Äther zur Kristallisation gebracht.

Die farblosen Kristalle werden abgesaugt und das Acetonitril umkristallisiert. Ausbeute : 1,4 g. Fp. : 144 bis 145 °C.

Beispiel 10

1-(2-Cyano-6-n-pentanoylaminophenoxy)-3-(2-äthylpentin-4-amino-3)-2-propanol-hydrochlorid

11 g (0,036 Mol) 1-(2-Cyano-6-n-pentanoylaminophenoxy)-3-chlor-2-propanol werden in 80 ml Äthanol unter Zugabe von 14,8 ml (0,11 Mol) 3-Äthylpentin-4-amin-3 wie in Beispiel 8 umgesetzt und aufgearbeitet. Die Reinigung der rohen Base wird ebenfalls über eine Kieselgelsäule durchgeführt. Die Base wird dann in wenig Acetonitril gelöst und mit ätherischer HCl angesäuert. Durch Zugabe von Äther und etwas Petroläther wird die Kristallisation des Hydrochlorids eingeleitet. Es werden 2,6 g Reinsubstanz isoliert. Fp. : 196 bis 198 °C.

Beispiel 11

1-(2-Cyano-6-n-hexanoylaminophenoxy)-3-(2-äthylpentin-4-amino-3)-2-propanol-hydrochlorid

11 g (0,033 Mol) 1-(2-Cyano-6-n-hexanoylaminophenoxy)-3-chlor-2-propanol werden in 70 ml Äthanol mit 12 ml (0,09 Mol) 3-Äthylpentin-4-amin-3 2,5 Stunden unter Rückfluß zum Sieden erhitzt. Aufarbeitung und Reinigung über eine Kieselgelsäule erfolgt wie in Beispiel 8. Das so gereinigte Amin wird in Äther gelöst, mit ätherischer HCl angesäuert und das Hydrochlorid mit Hexan ausgefüllt. Die Festsubstanz wird aus Acetonitril umkristallisiert. Ausbeute : 2,9 g. Fp. : 192 bis 193 °C.

B. Formulierungsbeispiele

1. Tabletten

| | |
|---|---|
| 1-(2-Cyano-4-n-hexanoylaminophenoxy)-3-(2-methylbutinyl-3-amino-2)-2-propanol | 40,0 mg |
| Maisstärke | 164,0 mg |
| sek. Calciumphosphat | 240,0 mg |
| Magnesiumstearat | 1,0 mg |
| | 445,0 mg |

Herstellung :
Die einzelnen Bestandteile werden intensiv miteinander vermischt und die Mischung in üblicher Weise granuliert. Das Granulat wird zu Tabletten von 445 mg Gewicht verpreßt, von denen jede 40 mg Wirkstoff enthält.

2. Gelatine-Kapseln

Der Inhalt der Kapsel setzt sich wie folgt zusammen :

| | |
|---|---|
| 1-(2-Cyano-4-n-hexanoylaminophenoxy-3-(2-methylbutinyl-3-amino-2)-2-propanol | 25,0 mg |
| Maisstärke | 175,0 mg. |
| | 200,0 mg |

Herstellung :
Die Bestandteile des Kapselinhalts werden intensiv miteinander vermischt und 200 mg-Portionen der Mischung werden in Gelatine-Kapseln geeigneter Größe abgefüllt. Jede Kapsel enthält 25 mg des Wirkstoffs.

3. Injektionslösung

Die Lösung wird aus folgenden Bestandteilen hergestellt :

| | |
|---|---|
| 1-(2-Cyano-4-n-octanoylamino-phenoxy)-3-2-methylbutinyl-3-amino-2)-2-propanol | 2,5 Teile |
| Natriumsalz der EDTA (Äthylendiamintetraessigsäure) | 0,2 Teil |
| dest. Wasser ad | 100,0 Teile |

Herstellung :

Der Wirkstoff und das EDTA-Salz werden in genügend Wasser gelöst und mit Wasser auf das gewünschte Volumen aufgefüllt. Die Lösung wird frei von suspendierten Partikeln filtriert und in 1 ccm-Ampullen unter aseptischen Bedingungen abgefüllt. Zuletzt werden die Ampullen sterilisiert und verschlossen. Jede Ampulle enthält 25 mg Wirkstoff.

4. Depotdragées

Kern :

| | |
|---|---|
| (−)-1-(2-Cyano-4-n-hexanoyl-phenoxy-3-(2-methylbutinyl-3-amino-2)-2-propanol | 25,0 g |
| Carboxymethylcellulose (CMC) | 295,0 g |
| Stearinsäure | 20,0 g |
| Celluloseacetatphthalat (CAP) | 40,0 g |
| | 380,0 g |

Herstellung :

Der Wirkstoff, die CMC und die Stearinsäure werden intensiv gemischt und die Mischung in üblicher Weise granuliert, wobei man eine Lösung des CAP in 200 ml eines Gemisches aus Äthanol/Äthylenacetat verwendet. Das Granulat wird dann zu 380 mg-Kernen verpreßt, die in üblicher Weise mit einer zuckerhaltigen 5 %igen Lösung von Polyvinylpyrrolidon in Wasser überzogen werden.

Jedes Dragée enthält 25 mg Wirkstoff.

5. Tabletten

| | |
|---|---|
| 1-(2-Cyano-4-n-heptanoylaminophenoxy)-3-(3-äthylpentinyl-4-amino-3)-2-propanol | 35,0 g |
| 2,6-Bis-(diäthanolamino)-4,8-dipeperdinopyrimido-[5,4-d]-pyrimidin | 75,0 g |
| Milchzucker | 164,0 g |
| Maisstärke | 196,0 g |
| kolloidale Kieselsäure | 14,0 g |
| Polyvinylpyrrolidon | 6,0 g |
| Magnesiumstearat | 2,0 g |
| lösliche Stärke | 10,0 g |
| | 500,0 g |

Anstelle des in diesem Beispiel genannten β-adrenolytisch wirksamen Stoffs kann auch die Substanz 1-(2-Cyano-4-n-hexanoylamino-phenoxy)-3-(2-methyl-butinyl-3-amino-2-propanol in gleicher Menge verwendet werden.

Herstellung :

Der Wirkstoff wird zusammen mit dem Milchzucker, der Maisstärke, der kolloidalen Kieselsäure und dem Polyvinylpyrrolidon nach intensiver Durchmischung in üblicher Weise granuliert, wobei man eine wäßrige Lösung der löslichen Stärke verwendet. Das Granulat wird mit dem Magnesiumstearat gemischt und zu 1 000 Tabletten von je 500 mg Gewicht verpreßt, die je 35 mg des ersten und 75 mg des zweiten Wirkstoffs enthalten.

**Ansprüche** (für die Vertragstaaten : BE, CH, DE, FR, IT, LI, LU, NL, SE)

1. Neue Verbindungen der allgemeinen Formel

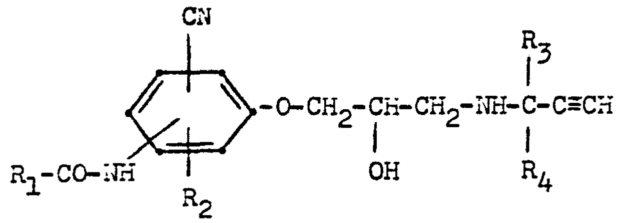

(I)

in der

$R_1$ einen geraden oder verzweigten Alkylrest mit 4 bis 20 C-Atomen

$R_2$ ein Wasserstoff- oder Halogenatom, eine Alkyl- oder Alkoxygruppe mit 1 bis 4 C-Atomen oder die zweiwertigen Gruppen $—CH = CH—CH = CH—$ oder $(—CH_2—)_n$ (n = ganze Zahl von 3 bis 5) mit Bindung der freien Valenzen in o-Stellung zueinander,

$R_3$ Wasserstoff oder einen Alkylrest mit 1 bis 3 C-Atomen

$R_4$ einen Alkyrest mit 1 bis 3 C-Atomen oder zusammen mit $R_3$ die Gruppe $(—CH_2—)_p$ (p = ganze Zahl von 4 bis 6),

bedeutet, und deren physiologisch verträgliche Säureadditionssalze.

2. Neue Verbindungen der allgemeinen Formel I, gemäß Anspruch 1, dadurch gekennzeichnet, daß $R_3$ und $R_4$ jeweils die Methylgruppe bedeuten, sowie deren physiologisch verträglichen Säureadditionssalze.

3. 1-(2-Cyano-4-n-hexanoylamino)-phenoxy-3-(2-methyl-butinyl-3-amino-2)-2-propanol und seine physiologisch verträglichen Säureadditionssalze.

4. Pharmazeutische Zubereitungen, enthaltend Substanzen der allgemeinen Formel I bzw. ihre physiologisch verträglichen Säureadditionssalze in Kombination mit üblichen Hilfs- und/oder Trägerstoffen.

5. Pharmazeutische Präparate nach Anspruche 4, enthaltend Substanzen der allgemeinen Formel I bzw. ihre physiologisch verträglichen Säureadditionssalze in Kombination mit anderen pharmazeutischen Wirkstoffen sowie üblichen Hilfs- und/oder Trägerstoffen.

6. Verfahren zur Herstellung von Verbindungen der allgemeinen Formel I gemäß Anspruch 1

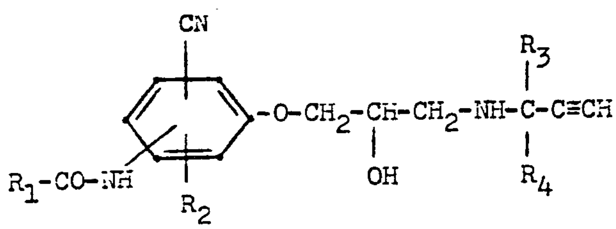

(I)

dadurch gekennzeichnet, daß man

a) eine Verbindung der allgemeinen Formel II

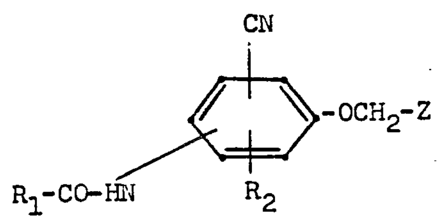

(II)

in der $R_1$ und $R_2$ wie in Formel I definiert sind und Z die Gruppe

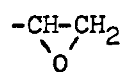

oder $—CHOH—CH_2—Hal$ (Hal = Halogen) bedeutet, mit einem Amin der allgemeinen Formel

$$NH_2—CR_3R_4—C{\equiv}CH \qquad (III)$$

in der $R_3$ und $R_4$ wie in Formel I definiert sind, umsetzt, oder daß man

b) ein Oxazolidin-Derivat der allgemeinen Formel IV

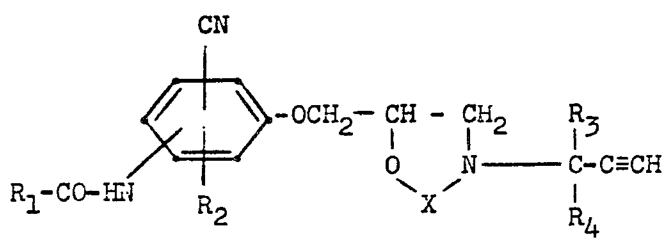

(IV)

in der $R_1$ bis $R_4$ wie in Formel I und X eine —CO—, —$CH_2$— oder —CH—(R)-Gruppe bedeutet, wobei R für Niederalkyl steht, definiert sind, hydrolysiert, oder daß man

c) eine Verbindung der allgemeinen Formel V

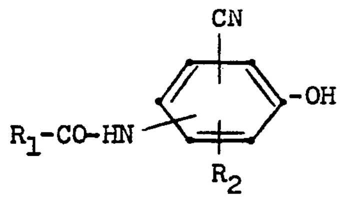

(V)

in welcher $R_1$ und $R_2$ wie in Formel I definiert sind, oder ein Salz dieser Verbindung, mit einem Azetidinolderivat der allgemeinen Formel

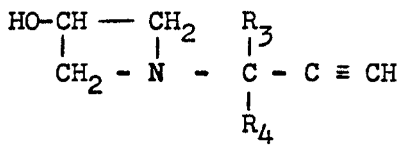

(VI)

in welcher $R_3$ und $R_4$ wie in Formel I definiert sind, umsetzt, und die nach einem der Verfahren a) bis c) erhaltenen Verbindungen gewünschtenfalls in ihre Säureadditionssalze überführt.

7. Verfahren zur Herstellung von optisch aktiven Verbindungen der allgemeinen Formel I, dadurch gekennzeichnet, daß man

a) von optisch aktiven Ausgangsmaterialien ausgeht, oder daß man

b) die nach einem der Verfahren des Anspruchs 6 erhaltenen Verbindungen durch Umsetzung mit optisch aktiven Hilfssäuren in ihre diastereomeren Salze überführt und letztere durch fraktionierte Kristallisation auftrennt.

**Ansprüche** (für den Vertragsstaat AT)

1. Verfahren zur Herstellung von Verbindungen der allgemeinen Formel I

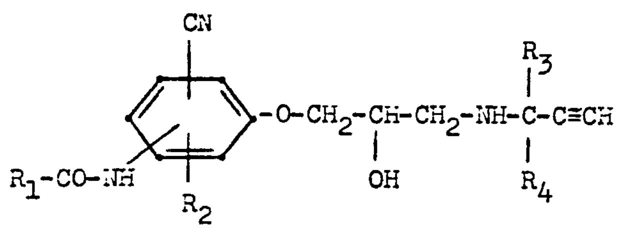

(I)

in der

$R_1$ einen geraden oder verzweigten Alkylrest mit 4 bis 20 C-Atomen

$R_2$ ein Wasserstoff- oder Halogenatom, eine Alkyl- oder Alkoxygruppe mit 1 bis 4 C-Atomen oder die zweiwertigen Gruppen —CH = CH—CH = CH— oder $(—CH_2—)_n$ (n = ganze Zahl von 3 bis 5) mit Bindung der freien Valenzen in o-Stellung zueinander,

$R_3$ Wasserstoff oder einen Alkylrest mit 1 bis 3 C-Atomen

$R_4$ einen Alkylrest mit 1 bis 3 C-Atomen oder zusammen mit $R_3$ die Gruppe $(—CH_2—)_p$ (p = ganze Zahl von 4 bis 6),

bedeutet, und deren physiologisch verträglichen Säureadditionssalzen, dadurch gekennzeichnet, daß man

a) eine Verbindung der allgemeinen Formel II

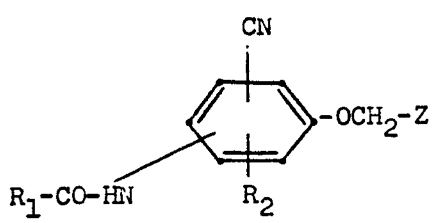

(II)

in der $R_1$ und $R_2$ wie in Formel I definiert sind und Z die Gruppe

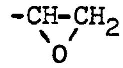

oder —CHOH—$CH_2$—Hal (Hal = Halogen) bedeutet, mit einem Amin der allgemeinen Formel

$$NH_2—CR_3R_4—C \equiv CH \qquad (III)$$

in der $R_3$ und $R_4$ wie in Formel I definiert sind, umsetzt, oder daß man

b) ein Oxazolidin-Derivat der allgemeinen Formel IV

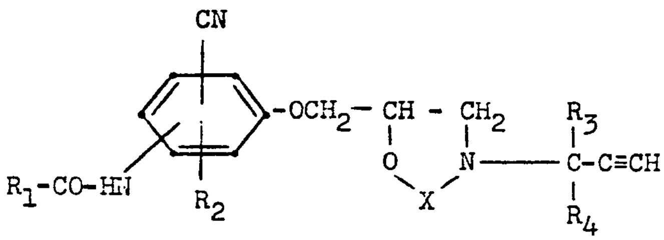

(IV)

in der $R_1$ bis $R_4$ wie in Formel I und X eine —CO—, —$CH_2$— oder —CH—(R)-Gruppe bedeutet, wobei R für Niederalkyl steht, definiert sind, hydrolysiert, oder daß man

c) eine Verbindung der allgemeinen Formel V

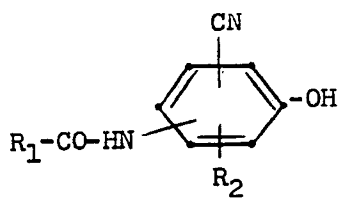

(V)

in welcher $R_1$ und $R_2$ wie in Formel I definiert sind, oder ein Salz dieser Verbindung, mit einem Azetidinolderivat der allgemeinen Formel

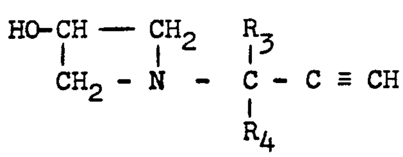

(VI)

in welcher $R_3$ und $R_4$ wie in Formel I definiert sind, umsetzt, und die nach einem der Verfahren a) bis c) erhaltenen Verbindungen gewünschtenfalls in ihre Säureadditionssalze überführt.

2. Verfahren zur Herstellung von optisch aktiven Verbindungen der allgemeinen Formel I, dadurch gekennzeichnet, daß man

a) von optisch aktiven Ausgangsmaterialien ausgeht, oder daß man

b) die nach einem der Verfahren des Anspruchs 1 erhaltenen Verbindungen durch Umsetzung mit optisch aktiven Hilfssäuren in ihre diastereomeren Salze überführt und letztere durch fraktionierte Kristallisation auftrennt.

**Claims** (for the Contracting States : BE, CH, DE, FR, IT, LI, LU, NL, SE)

1. New compounds of general formula

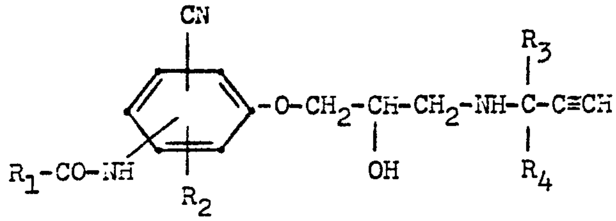

(I)

wherein

$R_1$ represents a straight-chained or branched alkyl group with 4 to 20 carbon atoms,

$R_2$ represents a hydrogen or halogen atom, an alkyl or alkoxy group with 1 to 4 carbon atoms or the divalent groups $-CH=CH-CH=CH-$ or $(-CH_2-)_n$ (wherein n = an integer from 3 to 5) with the free valencies bonded in the o-position relative to one another,

$R_3$ represents hydrogen or an alkyl group with 1 to 3 carbon atoms, and

$R_4$ represents an alkyl group with 1 to 3 carbon atoms or, together with $R_3$, represents the group $(-CH_2-)_p$ (wherein p = an integer from 4 to 6),

and the physiologically acceptable acid addition salts thereof.

2. New compounds of general formula I as claimed in claim 1, characterised in that $R_3$ and $R_4$ each represent a methyl group, and the physiologically acceptable acid addition salts thereof.

3. 1-(2-Cyano-4-n-hexanoylamino)-phenoxy-3-(2-methyl-butynyl-3-amino-2)-2-propanol and the physiologically acceptable acid addition salts thereof.

4. Pharmaceutical preparations containing substances of general formula I or the physiologically acceptable acid addition salts thereof in conjunction with conventional excipients and/or carriers.

5. Pharmaceutical preparations as claimed in claim 4, containing substances of general formula I or the physiologically acceptable acid addition salts thereof in conjunction with other pharmaceutical active substances and conventional excipients and/or carriers.

6. Process for preparing compounds of general formula I as claimed in claim 1,

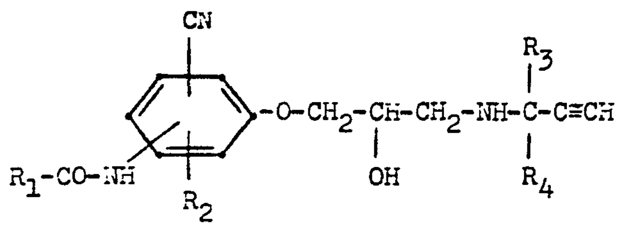

(I)

characterised in that

a) a compound of general formula II

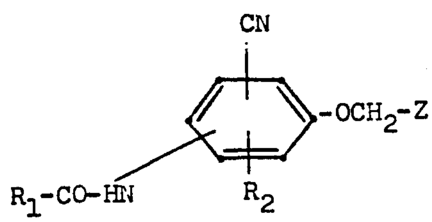

(II)

wherein $R_1$ and $R_2$ are defined as in formula I and Z represents the group

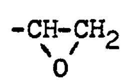

or $-CHOH-CH_2-Hal$ (wherein Hal = halogen), is reacted with an amine of general formula

$$NH_2-CR_3R_4-C\equiv CH \quad (III)$$

wherein $R_3$ and $R_4$ are defined as in formula I, or

b) an oxazolidine derivative of general formula IV

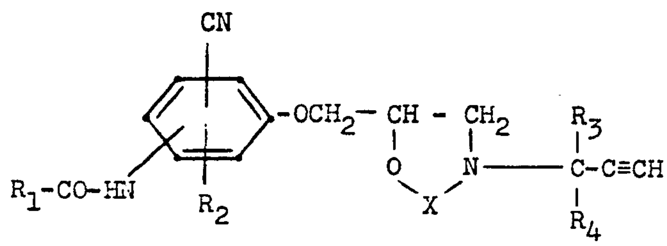

(IV)

wherein $R_1$ to $R_4$ are defined as in formula I and X represents a —CO—, —$CH_2$— or —CH—(R)-group (wherein R represents lower alkyl), is hydrolysed, or

c) a compound of general formula V

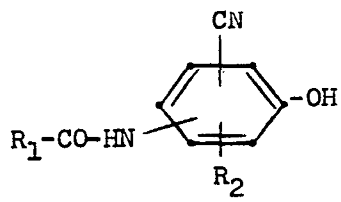

(V)

wherein $R_1$ and $R_2$ are defined as in formula I, or a salt of this compound, is reacted with an azetidinol derivative of general formula

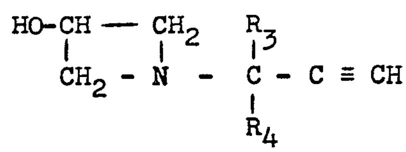

(VI)

wherein $R_3$ and $R_4$ are defined as in formula I, and, if desired, the compounds obtained according to one of the processes a) to c) are converted into the acid addition salts thereof.

7. Process for preparing optically active compounds of general formula I, characterised in that

a) optically active starting materials are used, or

b) the compounds obtained according to one of the processes in claim 6 are converted, by reacting them with optically active auxiliary acids, into the diastereomeric salts thereof and the latter are separated by fractional crystallisation.

**Claims** (for the Contracting State AT)

1. Process for the preparation of compounds of general formula I

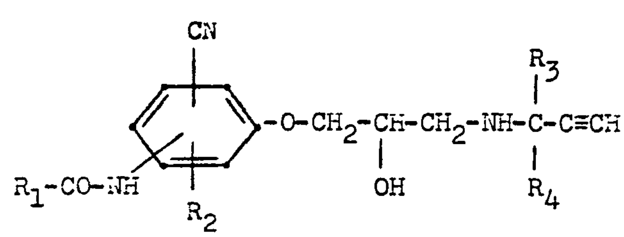

(I)

wherein

$R_1$ represents a straight-chained or branched alkyl group with 4 to 20 carbon atoms,

$R_2$ represents a hydrogen or halogen atom, an alkyl or alkoxy group with 1 to 4 carbon atoms or the

divalent groups $-CH=CH-CH=CH-$ or $(-CH_2-)_n$ (wherein n = an integer from 3 to 5) with the free valencies bonded in the o-position relative to one another,

$R_3$ represents hydrogen or an alkyl group with 1 to 3 carbon atoms, and

$R_4$ represents an alkyl group with 1 to 3 carbon atoms or, together with $R_3$, represents the group $(-CH_2-)_p$ (wherein p = an integer from 4 to 6),

and the physiologically acceptable acid addition salts thereof, characterised in that

a) a compound of general formula II

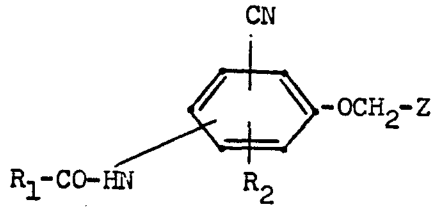

(II)

wherein $R_1$ and $R_2$ are defined as in formula I and Z represents the group

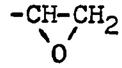

or $-CHOH-CH_2-Hal$ (wherein Hal = halogen), is reacted with an amine of general formula

$$NH_2-CR_3R_4-C\equiv CH \quad (III)$$

wherein $R_3$ and $R_4$ are defined as in formula I, or

b) an oxazolidine derivative of general formula IV

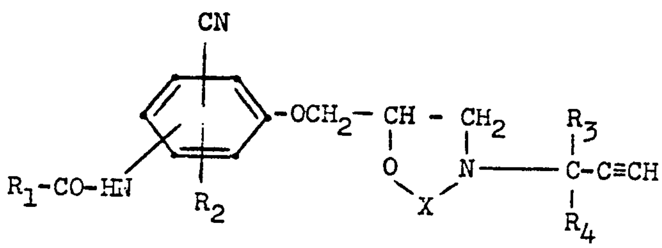

(IV)

wherein $R_1$ to $R_4$ are defined as in formula I and X represents a $-CO-$, $-CH_2-$ or $-CH-(R)$-group (wherein R represents lower alkyl), is hydrolysed, or

c) a compound of general formula V

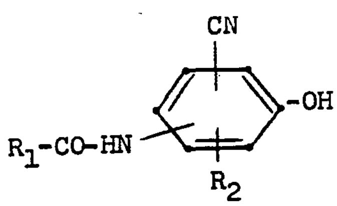

(V)

wherein $R_1$ and $R_2$ are defined as in formula I, or a salt of this compound, is reacted with an azetidinol derivative of general formula

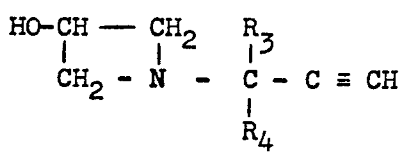

(VI)

wherein $R_3$ and $R_4$ are defined as in formula I, and, if desired, the compounds obtained according to one of the processes a) to c) are converted into the acid addition salts thereof.

2. Process for preparing optically active compounds of general formula I, characterised in that

a) optically active starting materials are used, or

b) the compounds obtained according to one of the processes in claim 1 are converted, by reacting them with optically active auxiliary acids, into the diastereomeric salts thereof and the latter are separated by fractional crystallisation.

**Revendications** (pour les Etats contractants : BE, CH, DE, FR, IT, LI, LU, NL, SE)

1. Nouveaux composés de formule générale

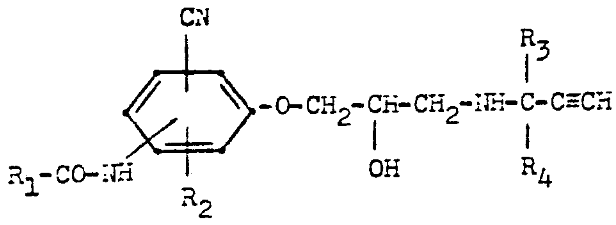

(I)

dans laquelle

$R_1$ représente un radical alcoyle rectiligne ou ramifié avec 4 à 20 atomes de C

$R_2$ représente un atome d'hydrogène ou d'halogène, un groupe alcoyle ou alcoxy avec 1 à 4 atomes de C ou les groupes bivalents $-CH=CH-CH=CH-$ ou $(-CH_2-)_n$ (n = nombre entier de 3 à 5) avec liaison des valences libres en position o l'une par rapport à l'autre,

$R_3$ représente un atome d'hydrogène ou un radical alcoyle avec 1 à 3 atomes de C

$R_4$ représente un radical alcoyle avec 1 à 3 atomes de C ou ensemble avec $R_3$ le groupe $(-CH_2-)_p$ (p = nombre entier de 4 à 6),

et leurs sels d'addition d'acides physiologiquement supportables.

2. Nouveaux composés de formule générale I selon la revendication 1, caractérisés en ce que $R_3$ et $R_4$ représentent chacun le groupe méthyle, ainsi que leurs sels d'addition d'acides physiologiquement supportables.

3. Le 1-(2-cyano-4-n-hexanoylamino)-phénoxy-3-(2-méthyl-butynyl-3-amino-2)-2-propanol et ses sels d'addition d'acides physiologiquement supportables.

4. Préparations pharmaceutiques contenant des substances de formule générale I ou leurs sels d'addition d'acides physiologiquement supportables en combinaison avec des adjuvants et/ou excipients habituels.

5. Préparations pharmaceutiques selon la revendication 4, contenant des substances de formule générale I ou respectivement leurs sels d'addition d'acides physiologiquement supportables en combinaison avec d'autres substances actives pharmaceutiques ainsi que des excipients et/ou adjuvants habituels.

6. Procédé pour la préparation de composés de formule générale I selon la revendication 1,

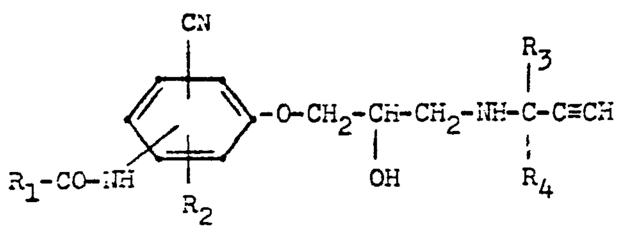

(I)

caractérisé en ce que

a) on fait réagir un composé de formule générale II

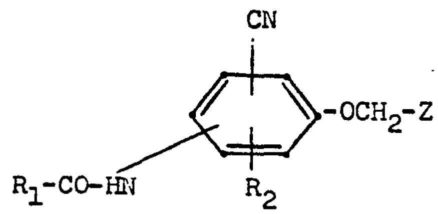

(II)

dans laquelle $R_1$ et $R_2$ sont définis comme dans la formule I et Z représente le groupe

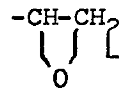

ou —CHOH—$CH_2$—hal (hal = halogène), avec une amine de formule générale

$$NH_2—CR_3R_4—C \equiv CH \qquad (III)$$

dans laquelle $R_3$ et $R_4$ sont définis comme dans la formule I, ou en ce que

b) on hydrolyse un dérivé d'oxazolidine de formule générale IV

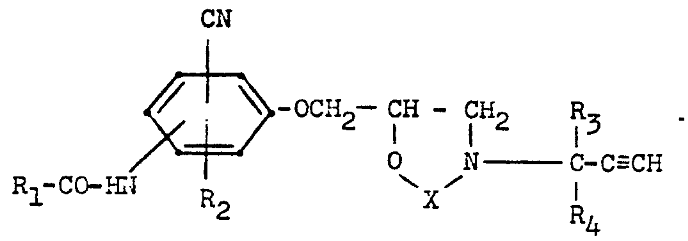

(IV)

dans laquelle $R_1$ à $R_4$ sont définis comme dans la formule I et X représente un groupe —CO—, —$CH_2$— ou —CH—(R), R représentant alcoyle inférieur, ou en ce que

c) on fait réagir un composé de formule générale V

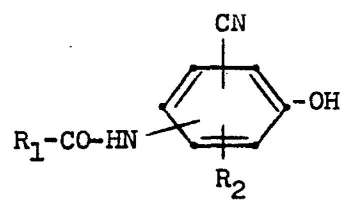

(V)

dans laquelle $R_1$ et $R_2$ sont définis comme dans la formule I, ou un sel de ce composé, avec un dérivé d'azétidinol de formule générale

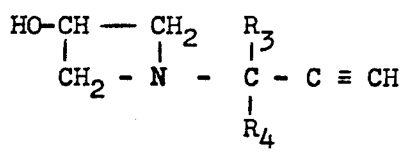

(VI)

dans laquelle $R_3$ et $R_4$ sont définis comme dans la formule I, et en ce que l'on transforme si on le désire les composés obtenus selon l'un des procédés a) à c) en leurs sels d'addition d'acides.

7. Procédé pour la préparation de composés de formule générale I optiquement actifs, caractérisé en ce que

a) on part de matières de départ optiquement actives, ou en ce que

b) on transforme les composés obtenus selon l'un des procédés de la revendication 6, par réaction avec des acides adjuvants optiquement actifs, en leurs sels diastéréoisomères et on sépare ces derniers par cristallisation fractionnée.

**Revendications** (pour l'Etat contractant AT)

1. Procédé pour la préparation de composés de formule générale I

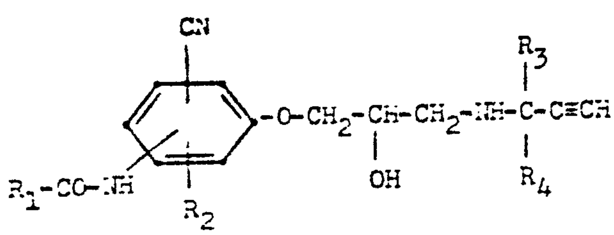

(I)

dans laquelle

$R_1$ représente un radical alcoyle rectiligne ou ramifié avec 4 à 20 atomes de C,

$R_2$ représente un atome d'hydrogène ou d'halogène, un groupe alcoyle ou alcoxy avec 1 à 4 atomes de C ou les groupes bivalents $—CH=CH—CH=CH—$ ou $(—CH_2—)_n$ (n = nombre entier de 3 à 5) avec liaison des valences libres en position o l'une par rapport à l'autre,

$R_3$ représente l'hydrogène ou un radical alcoyle avec 1 à 3 atomes de C

$R_4$ représente un radical alcoyle avec 1 à 3 atomes de C ou ensemble avec $R_3$ le groupe $(—CH_2—)_p$ (p = nombre entier de 4 à 6),

et de leurs sels d'addition d'acides physiologiquement supportables, caractérisé en ce que

a) on fait réagir un composé de formule générale II

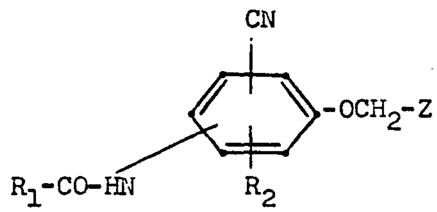

(II)

dans laquelle $R_1$ et $R_2$ sont définis comme dans la formule I et Z représente le groupe

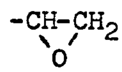

ou $—CHOH—CH_2—$hal (hal = halogène), avec une amine de formule générale

$$NH_2—CR_3R_4—C\equiv CH \qquad (III)$$

dans laquelle $R_3$ et $R_4$ sont définis comme dans la formule I, ou en ce que

b) on hydrolyse un dérivé d'oxazolidine de formule générale IV

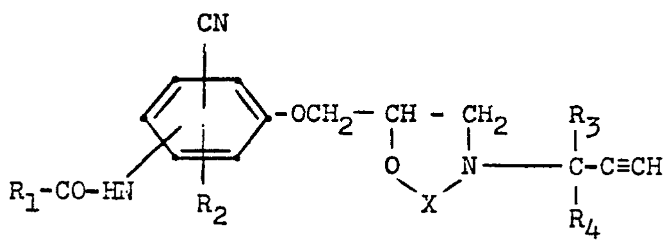

(IV)

dans laquelle $R_1$ et $R_4$ sont définis comme dans la formule I et X représente un groupe $—CO—$, $—CH_2—$ ou $—CH—(R)$, R représentant alcoyle inférieur, ou en ce que

c) on fait réagir un composé de formule générale V

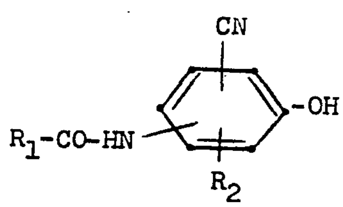

(V)

dans laquelle $R_1$ et $R_2$ sont définis comme dans la formule I, ou un sel de ce composé, avec un dérivé d'azétidinol de formule générale

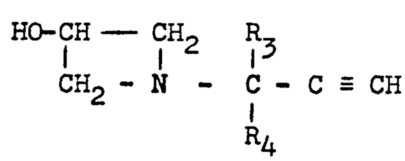

(VI)

dans laquelle $R_3$ et $R_4$ sont définis comme dans la formule I, et en ce que l'on transforme si on le désire les composés obtenus selon l'un des procédés a) à c) en leurs sels d'addition d'acide.

2. Procédé pour la préparation de composés de formule général I optiquement actifs, caractérisé en ce que

a) on part de matières de départ optiquement actives, ou en ce que

b) on transforme les composés obtenus selon l'un des procédés de la revendication 1 par réaction avec des acides adjuvants optiquement actifs en leurs sels diastéréoisomères et on sépare ces derniers par cristallisation fractionnée.